# EUROPEAN PATENT SPECIFICATION

(11) **EP 2 076 246 B1**
(45) Date of publication and mention of the grant of the patent: **01.03.2017**
(21) Application number: 07852671.2
(22) Date of filing: 11.10.2007
(51) Int. Cl.: A61K 9/14, A61K 9/00, A61K 9/10, A61K 9/70, A61L 26/00

(54) **FORMATION OF MEDICALLY USEFUL GELS COMPRISING MICROPOROUS PARTICLES AND METHODS OF USE**
FORMIERUNG MEDIZINISCH NÜTZLICHER GELE MIT MIKROPORÖSEN PARTIKELN UND VERWENDUNGSVERFAHREN DAFÜR
FORMATION DE GELS MÉDICALEMENT UTILES COMPRENANT DES PARTICULES MICROPOREUSES ET LEURS PROCÉDÉS D'UTILISATION

(30) Priority: 13.10.2006 US 580372
(43) Date of publication of application: 08.07.2009
(73) Proprietor: MEDAFOR, INCORPORATED, Minneapolis MN 55430 (US)
(72) Inventor: DRAKE, James F., Minneapolis MN 55414 (US); GRONDA, Ann, New Brighton MN 55112 (US)
(74) Representative: Ipside
(86) International application number: PCT/US2007/021732
(87) International publication number: WO 2008/048468

(56) References cited:
- WO-A1-01/64148
- US-A- 6 060 461
- US-A- 6 060 461
- US-A1- 2001 047 187
- US-A1- 2003 108 511
- US-A1- 2003 108 511

## Description

### BACKGROUND OF THE INVENTION

### 1. Field of the Invention

The present invention relates to the treatment of wounds or trauma, or protection of wounds or trauma resulting from intended medical treatment such as surgery. Compositions are described that are applied to the areas of the wound or trauma and methods for application of the compositions are described. Treatments include application to internal organs and tissue as part of enhancing recovery from surgery.

### 2. Background of the Art

Adhesions are fibrous bands of scar-like tissue adhering to internal organs, bones, or tissues, anchoring them to each other or adjacent structures. These adhesions can form following surgical procedures that damage or irritate the peritoneal tissues lining the organs of the abdominal cavity. In many cases the fibrous bands can bind, twist or otherwise interfere with the affected organs. The adhesions often form during a natural, but prolonged healing process after tissues or organs have been traumatized during medical procedures. Such traumatized tissue can adhere to surface which they ordinarily would not attach to during this recovery process, and these attachments can create tensions between tissues and organs that affect the patient.

A number of products and procedures have been proposed to minimize the formation of adhesions. Specialized surgical techniques such as laparoscopy or microsurgery seek to minimize trauma to the internal organs in an attempt to limit the formation of adhesions.

Drug treatments using anti-inflammatory agents, prostaglandins, and specialized antibody formulations have been used with limited success. These drug regimens attempt to block the complex inflammatory process that follows injury and healing to perhaps direct the healing process toward the growth of healthy peritoneal tissue rather than formation of fibrous scar tissue.

U.S. Patent No. 6,949,114 (Milo et al.) discloses systems and methods that convey a closure material into a catheter to seal a puncture site in a blood vessel. The closure material comprises a mixture of first and second components which, upon mixing, undergo a reaction to form a solid closure material composition. The systems and methods assure ease of delivery and effective mixing of the components to create an in situ barrier at the puncture site. A material composition physically forms a mechanical barrier (see FIG. 17), which can also be characterized as a hydrogel.

U.S. Patent No. 6,083,524 (Sawnhey et al.) describes novel polymer compositions for forming hydrogels for medical adhesive compositions. Water-soluble macromers including at least one hydrolysable linkage formed from carbonate or dioxanone groups, at least one water-soluble polymeric block, and at least one polymerizable group, and methods of preparation and use thereof are described. The macromers are preferably polymerized using free radical initiators under the influence of long wavelength ultraviolet light or visible light excitation. Biodegradation occurs at the linkages within the extension oligomers and results in fragments which are non-toxic and easily removed from the body. The macromers can be used to encapsulate cells, deliver prophylactic, therapeutic or diagnostic agents in a controlled manner, plug leaks in tissue, prevent adhesion formation after surgical procedures, temporarily protect or separate tissue surfaces, and adhere or seal tissues together.

U.S. Patent No. 5,410,016 (Hubbell et al.) discloses biocompatible, biodegradable macromers which can be polymerized to form hydrogels. The macromers are block copolymers that include a biodegradable block, a water-soluble block with sufficient hydrophilic character to make the macromer water-soluble, and one or more polymerizable groups. The polymerizable groups are separated from each other by at least one degradable group, Hubbell specifically discloses using polyhydroxy acids, such as polylactide, polyglycolide and polycaprolactone as the biodegradable polymeric blocks. One of the disclosed uses for the macromers is to plug or seal leaks in tissue.

Other hydrogels have been described, for example, in U.S. Patent No. 4,938,763 (Dunn et al.); U.S. Patent Nos. 5,100,992 and 4,826,945 (Cohn et al.); U.S. Patent Nos. 4,741,872 and 5,160,745 (De Luca et al.); U.S. Patent No. 5,527,864 (Suggs et al.); and U.S. Patent No. 4,511,478 (Nowinski et al.). Methods of using such polymers are described in U.S. Patent No. 5,573,934 (Hubbell et al.) and PCT WO 96/29370 (Focal).

US 2003/108511 A1 relates to a system for coating a tissue comprising two different components stored in an applicator system displaying two separate compartments for said two different components. When mixed together by spraying on the tissue, the two components react to form a crosslinked hydrogel. The composition comprises macroparticles entrapped in a hydrogel matrix.

Many references disclose using homopolymers and copolymers including carbonate linkages to form solid medical devices, such as sutures, suture coatings and drug delivery devices (see, for example, U.S. Patent No. 3,301,824 (Hostettler et al.); U.S. Patent No. 4,243,775 (Rosensaft et al.); U.S. Patent No. 4,429,080 (Casey et al.); U.S. Patent No. 4,716,203 (Casey et al.); U.S. Patent No. 4,857,602 (Casey et al.); U.S. Patent No. 4,882,168 (Casey); EP 0 390 860 B1 (Boyle et al.); U.S. Patent No. 5,066,772 (Tang et al.); U.S. Patent No. 5,366,756 (Chesterfield et al.); U.S. Patent No. 5,403,347 (Roby et al.); and U.S. Patent No. 5,522,841 (Roby et al.).

Barrier products are administered following surgery to protect and separate the organs with the goal of preventing adhesions. Over the years, a variety of barrier materials such as silk, metal foils, animal membranes, oils and plastic films have been used as adhesion preventives. In all cases it was hoped that keeping the organs separated until healing of the injured surfaces occurred would prevent or minimize adhesion formation. Most of these products have been abandoned in favor of newer barrier formulations consisting of thin films or gels that are easier to apply. Some of the more successful products are:
Seprafilm™, from Genzyme Corporation, is a composite film formed from sodium hyaluronate and carboxymethycellulose. The film slowly dissolves and is eventually eliminated from the body in about 30 days.
Hyskon™, from Medisan Pharmaceuticals, is a 70% solution of dextran in water that lubricates tissue and is absorbed in one week.
Flo-Gel™, produced by Alliance Pharmaceutical, is a sterile gel of Poloxamer 407, a block co-polymer of polyoxyethylene and polyoxypropylene. It is slowly eliminated from the body
Interceed™, from Ethicon Corporation, is a special grade of oxidized regenerated cellulose. It is absorbed in about 28 days.

All of these products seek to produce a soft, compliant barrier for separating the organs for 3 to 5 days until healing is complete. It is desirable that the barriers not remain in the body after healing is complete. Although many products have been used with some success, none is completely successful. Semi-solid gels and plastic films or fibers may not cover all of the exposed surfaces, small crevices or narrow spaces between tissues may not receive a protective film, or difficulty in applying the material may limit the effectiveness of the barrier. Less viscous fluid barriers, such as crystalloid solutions or weak gels, may cover surfaces well, but reabsorb before the healing process is complete. Clearly there is a need for new approaches and improved methods for creating and applying adhesion barriers.

### SUMMARY OF THE INVENTION

The present invention relates to a composition for use in the treatment of wounds or trauma of a surface of tissue of a patient, the composition being applied to the surface of tissue so that both a) a gel-forming composition comprising a solution, suspension, dispersion or emulsion and b) dry porous dextran or starch microparticles having pore sizes for molecular weight cutoff of between 5,000 Daltons and 200,000 Daltons are applied to the surface.

Compositions and methods for using the gel-forming properties of microporous particles to create useful formulations combine two free-flowing materials to produce a hydrogel mass are disclosed. The fluid materials comprise first dry microporous particles (preferably as an aerosol) that may contain additional agents, and a second composition of a fluid material which is an aqueous solution, suspension, dispersion or emulsion, preferably of one or more high molecular weight polymers capable of forming a hydrogel upon further concentration and/or reaction. The gels or hydrogels can be preferably formed on a surface by spraying the two compositions as fluids together in the proper ratio onto the surface, or by alternately applying one fluid and then the other to the surface (in either order). The extremely rapid formation of the gels when aerosols of microporous particles of the proper composition are combined *in situ* with said solutions, dispersions or emulsions allows the gels to be easily formed on vertical surfaces or in difficult to reach irregular spaces, such as within cavities of patients. The formation of the hydrogels *in situ* can circumvent some of the problems that arise when using existing products and allows gels to be applied to areas that may be difficult or impossible to reach with a preformed gel or film.

The porous microparticles of the invention are selected from dextran (Sephadex™, Pharmacia, Inc)) or starch (Microporous Polysaccharide Hemospheres™ (MPH), Medafor, Inc). Porous particles of the proper composition, when exposed to aqueous solutions of high molecular weigh materials, will rapidly imbibe water and concentrate the large molecules on the surface of the particles. This concentration can result in the formation of a thick viscous gel or hydrogel at the particle surface. For instance, application of MPH particles to a bleeding wound will induce the formation of a thick gel by concentration of blood proteins and cells effectively controlling the bleeding. Such use of microporous particles as hemostatic agents is described in US Patent 6,060,461. This phenomenon is not limited to the components of blood. It has been found that many polymer solutions will form gels when exposed to dry microporous particles of the current invention. Particles capable of rapidly forming gels from such solutions include Medafor's MPH starch particles, Sephadex™ G-50 dextran particles, and BioRad P60 polyacrylamide particles. For internal applications, the degradable starch particles are preferred while for topical applications any of the above may be used. Particles can be amended to include materials such as calcium chloride, thrombin, dyes for visualization, protein cross-linking agents, medicinal materials such as antibiotics or anti-inflammatory agents, or wound healing peptides. Useful polymer solutions include, but are not limited to, 0.5% sodium alginate, citrated blood plasma, 25% human serum albumin available as a sterile product for intravenous use, sodium hyaluronic acid, human fibrinogen, carboxymethycellulose, hydroxypropylcellulose, and polyvinylpyrollidone.

Other different types of microporous particles may include anion exchanger based on silica gel (Adsorbex™-SAX, Cat. No. 19845; Merck, Darmstadt, G.); cation exchanger (Adsorbex™-SCX, Cat. No. 19846), reversed-phase RP8 (Cat. No. 9362), and the like.

### DETAILED DESCRIPTION OF THE INVENTION

Hydrogels are formed by creating bridges between and within polymer chains through the attachment of small bridging molecules to the functional moieties of the polymer backbone, a process known as cross-linking. The structural integrity of conventional hydrogels is based upon the covalent chemistry used for the cross-linking, which typically requires catalysts to facilitate the reactions in a timely fashion. The presence of catalysts impedes the medical use of hydrogels, especially in surgical applications, because they are potentially injurious to surrounding tissues. Thus, hydrogels that can be polymerized rapidly without the use of chemical cross-linking catalysts as disclosed in U.S. Patent No. 6,949,590 (Ratner et al.) are desirable.

Typically hydrogels may comprise gels or hydrogels formed by a hydrophilic polymer which, as a result of hydrogen bond formation or covalent bonds, has pronounced water-binding characteristics. The hydrophilic polymer can absorb at least its own weight in water. Preferably it can contain at least 50%, at least 60% or 75-99.5 wt%, in particular 90-99 wt % of water, based on the sum of polymer and water. The structure of the hydrophilic polymer must be such that the bonds remain intact up to a temperature of about 80 degree C, preferably up to at least 90°C. Optionally, a hydrophilic organic solvent such as an alcohol, acetone, glycol, glycerol or polyglycol may also be present, but preferably less than 20 wt %, in particular less than 5 wt %, of this is present, based on the water.

The hydrophilic polymer may be, by way of non-limiting examples, a polymer or copolymer of acrylic acid or (meth)acrylic acid or a salt thereof, alkyl or hydroxyalkyl (meth)acrylate, (meth)acrylamide, vinylpyrrolidone and/or vinyl alcohol, polyethylene glycol, polyethylene oxide, or an optionally cross-linked, optionally modified polysaccharide such as starch, cellulose, guar gum, xanthan and other polysaccharides and gums and derivatives thereof such as hydroxyethyl-, hydroxypropyl- or carboxymethyl-cellulose or -starch. Polysaccharides modified with (poly)acrylates are likewise suitable. Preferably, the hydrophilic polymer contains hydroxyalkyl (meth)acrylate units and/or (meth)acrylamide units, where the (meth)acrylamide groups may be N-alkylated or N-hydroxyalkylated. Examples of monomers of which the hydrophilic polymer may be composed are, in particular, hydroxyethyl methacrylate and also hydroxypropyl methacrylate, dihydroxypropyl methacrylate, hydroxyethoxyethyl methacrylate, also ethoxylated analogues thereof, di(hydroxyethyl)aminoethyl methacrylate, methacrylamide, N,N-dimethylmethacrylamide, N-hydroxyethylmethacrylamide, N,N-bis(hydroxyethyl)methacrylamide, methacrylic acid, methyl methacrylate and the corresponding acrylates and acrylamides, N-vinylpyrrolidone and the like. They may be crosslinked with, for example, 0.1-2 wt % of ethylene dimethacrylate, oxydiethylene dimethacrylate, trimethylolpropane trimethacrylate, N,N-methylenebismethacrylamide and the like. Also suitable is a crosslinked polymer containing carbamoyl and carboxyl units having the formula >C(CONH₂)-C(COOH)<, which can be obtained by a polymer with maleic anhydride groups such as a vinyl methyl ether/maleic anhydride copolymer crosslinked with C₉H₁₈ chains being treated with ammonia.

The gelable material is preferably at least one ingredient selected from the group consisting of thrombin, albumin-fibrinogen, hyaluronan, cellulosic polymer, acrylic polymer, hydrolyzable polymer and crosslinkable polymer. The hydrophilic components may be further described as including at least 50%, at least 75% or at least 80% by weight of serum, serum fractions, solutions of albumin, gelatin, fibrinogen, and serum proteins. In addition, water soluble derivatives of hydrophobic proteins can be used. Examples include solutions of collagen, elastin, chitosan, and hyaluronic acid. In addition, hybrid proteins with one or more substitutions, deletions, or additions in the primary structure or as pendant structures may be used. Both the first composition and the second composition preferably are applied by spraying.

The gel or hydrogel is thus preferably in a semisolid state, so that liquid water cannot leak out even at elevated temperature. At the same time it has virtually the same high heat capacity as water.

The microporous particles may be any porous particle having an average (weight average or number average) size of about 0.25 to 1000 micrometers. The particles may generally have a size of from about 1 to 1000 micrometers, or 1 to 500 micrometers, but the size may be varied by one ordinarily skilled in the art to suit a particular use or type of patient and depending on the ability of a carrier to support the particles with their optional selection of sizes. Examples of specific materials useful in the practice of the present invention comprise porous materials from within the classes of polysaccharides, cellulosics, polymers (natural and synthetic), inorganic oxides, ceramics, zeolites, glasses, metals, and composites. Preferred materials are of course non-toxic and are provided as a sterile supply. The polysaccharides are preferred because of their ready availability and modest cost. The porous particulate polysaccharides may be provided as starch, cellulose and/or pectins, and even chitin may be used (animal sourced from shrimp, crab and lobster, for example). Glycosaccharides or glycoconjugates which are described as associations of the saccharides with either proteins (forming glycoproteins, especially glycolectins) or with a lipid (glycolipid) are also useful. These glycoconjugates appear as oligomeric glycoproteins in cellular membranes. In any event, all of the useful materials must be porous enough to allow blood liquid and low molecular weight blood components to be adsorbed onto the surface and/or absorbed into the surface of the particles. Porosity through the entire particle is often more easily achieved rather than merely etching the surface or roughening the surface of the particles. The microporous particles preferably comprise at least 5%, at least 8%, at least 10% or at least 15% by weight of the total solids (i.e., not inclusive of water or solvent) in the composition applied according to the present technology.

Ceramic materials may be provided from the sintering, or sol-gel condensation or dehydration of colloidal dispersions of inorganic oxides such as silica, titanium dioxide, zirconium oxide, zinc oxide, tin oxide, iron oxide, cesium oxide, aluminum oxide and oxides of other metal, alkaline earth, transition, or semimetallic chemical elements, and mixtures thereof. By selection of the initial dispersion size or sol size of the inorganic oxide particles, the rate of dehydration, the temperature at which the dehydration occurs, the shear rate within the composition, and the duration of the dehydration, the porosity of the particles and their size can be readily controlled according the skill of the ordinary artisan.

With regard to cellulosic particles, the natural celluloses or synthetic celluloses (including cellulose acetate, cellulose butyrate, cellulose propionate, etc.) may be exploded or expanded according to techniques described in U.S. Pat. No. 5,817,381 and other cellulose composition treating methods described therein which can provide porous particles, fibers and microfibers of cellulose based materials. Where the porous materials, whether of cellulose or other compositions, have a size which may be too large for a particular application, the particles may be ground or milled to an appropriate size. This can be done by direct mortar and pestle milling, ball milling, crushing (as long as the forces do not compress out all of the porosity), fluidized bed deaggregation and size reduction, and any other available physical process. Where the size of the raw material should be larger than the particle size provided, the smaller particles may be aggregated or bound together under controlled shear conditions with a binder or adhesive until the average particle size is within the desired range.

Porosity may be added to many materials by known manufacturing techniques, such as 1) codispersion with a differentially soluble material, and subsequent dissolution of the more soluble material, 2) particle formation from an emulsion or dispersion, with the liquid component being evaporated or otherwise removed from the solid particle after formation, 3) sintering of particles so as to leave porosity between the sintered or fused particles, 4) binding particles with a slowly soluble binder and partially removing a controlled amount of the binder, 5) providing particles with a two component, two phase system where one component is more readily removed than another solid component (as by thermal degradation, solubilization, decomposition, chemical reaction such as, chemical oxidation, aerial oxidation, chemical decomposition, etc.), and other known process for generating porosity from different or specific types of compositions and materials. Where only surface porosity is needed in a particular clot promoting format, surface etching or abrasion may be sufficient to provide the desired surface porosity.

The dry porous microparticles of the invention are dextran beads which are available as Sephadex™ beads from Pharmacia Labs. These are normally used in surgery as an aid to debridement of surfaces to help in the removal of damaged tissue and scar tissue from closed wounds. The application of this type of porous bead (and the other types of porous beads, such as those formed from crosslinked starch) to open wounds with blood thereon has been found to promote hemostasis, speeding up the formation of clots, and reducing blood loss and the need for continuous cleaning of the wound area.

The porous micropoarticles of the present invention are cross-linked dextran (poly[beta-1,6-anhydroglucose]) or starch (poly{alpha-1,4-anhydroglucose]). Dextran is a high molecular weight, water-soluble polysaccharide. It is not metabolized by humans, is non-toxic, and is well tolerated by tissue in most animals, including most humans. There has even been extensive use of solubilized dextrans as plasma substitutes. Similarly, beads prepared by cross linking starch with epichlorohydrin are useful as hemostatic agents and are well tolerated by tissue. The starch particles are enzymatically degraded by tissue alpha-amylases and rapidly removed from the wound site. The Sephadex™ beads specifically mentioned in the description of particularly useful polysaccharides comprise dextran crosslinked with epichlorihydrin. These beads arc available in a variety of bead sizes (e.g., 10 to 100 micrometers) with a range of pore sizes. It is believed that pore sizes on the order of from 5 to 75% of volume may be commercially available and can be expanded to from 5 to 85% by volume or manufactured with those properties from amongst the type of beads described above. The sizes of the pores may also be controlled to act as molecular sieves, the pore size being from 0.5% or 1 to 15% of the largest diameter of the particles or beads. The Sephadex™ beads are promoted as having controlled pore sizes for molecular weight cutoff of molecules during use as a sieve, e.g., with cutoff molecular diameters being provided at different intervals between about 5,000 Daltons and 200,000 Daltons. For example, there are cutoff values specifically for molecular weight sizes of greater than 75,000 Daltons. This implies a particle size of specifically about 10 to 40 microns. These beads will rapidly absorb water, swelling to several times their original diameter and volume (e.g., from 5 to as much as twenty times their volume). Similar technology can be used to produce cross linked starch beads with properties similar to the Sephadex™ particles. Other soluble polysaccharides such as sodium alginate or chitosan can be used to prepare cross linked beads with controlled porosity and size.

The porosity of the particles may vary according to specific designs of the final use and compositions. In a non-limiting estimate, it is believed that the effective volume of the particles should comprise from at least 2% to as much as 75% by volume of voids. More precisely, to assure a balance of structural strength for the particles and sufficient absorbency, a more preferred range would be about 5-60%, or 8-40% by volume as void space.

The two-component compositions of the present invention may be separately contained and then separately applied by spray or other physical application (laminar flow application, wipe, drip and wipe, swab, etc, although a spray is preferred for speed and relative uniformity of application). The spray may be liquid or gaseous supported. The rate of application (both with regard to total application time, speed and volume) may be controlled. Alternatively, the two materials may be mixed together prior to containment, or mixed just before the time of application. These and other features will be further appreciated after a reading of the following, non-limiting examples.

### Examples

### Example 1.

Ten grams of starch particles (MPH, Medafor, Inc) were combined with 10 ml of a solution containing 0.9% calcium chloride and .01% Evans Blue Dye. The resulting slurry was mixed, dried, and ground with a mortar and pestle to pass through a 100-micron screen. The resulting light blue powder was loaded into a carbon dioxide-powered spray applicator (Genuine Innovations, Tucson, AZ) capable of producing a fine mist of dry powders or liquids. A solution of 0.5% sodium alginate was loaded into a second spray applicator. The MPH powder was sprayed onto the surface of piece of fresh beef liver to form a dry visible layer. The 0.5% sodium alginate solution was then sprayed until the surface appeared wet. The wet surface was then re-sprayed with the MPH particles, followed by an additional layer of sodium alginate. Diffusion of calcium from the MPH particles resulted in the formation of an adherent, translucent coating of calcium alginate and starch particles on the surface of the tissue.

### Example 2.

MPH particles were loaded into a sprayer and applied to the surface of fresh beef liver. The particles stuck to the moist surface and accumulated as a white, dry layer. Human serum albumin (25%, sterile solution, ZLB Bioplasma™ AG) was loaded into another spray unit and sprayed onto the MPH layer until the surface appeared glossy and moist. The procedure was repeated and a final coating of MPH was applied until the surface appeared dry. The resulting film was examined and found to be a thick gel that adhered to the liver tissue.

### Example 3.

Five grams of the MPH particles were mixed with 20,000 units of lyophilized bovine thrombin (Sigma Chemical, St Louis), ground lightly in a mortar, and screened through a 100-micron sieve. The particles were loaded into a sprayer and applied to the surface of fresh beef liver. Human serum albumin (25%, sterile solution, ZLB Bioplasma AG) to which was added 6 mg per ml of bovine fibrinogen was then sprayed on the MPH coating. Thrombin diffusing from the MPH particles rapidly polymerized the fibrinogen to form a fibrin film, which entrapped the MPH particles. The resulting coating was strongly adhered to the tissue surface.

### Example 4.

A 40 kg pig was anesthetized and prepared for surgery. A midline laparotomy was preformed and the internal bowels exposed. Ten ml of blood was drawn and centrifuged to yield about 5 ml of citrated plasma. The plasma was loaded into a spray applicator. The MPH powder from Example 1 was then sprayed on the exposed intestine of the pig until a dry surface was obtained. Plasma was then sprayed onto the MPH coating to lightly wet the surface. An adherent gel formed. The process was repeated to create an additional layer of MPH/plasma. A firm gel of serum and MPH particles was formed. Within about five minutes, calcium diffusing from the MPH particles had initiated clotting of the plasma to form a firm, opaque layer on the bowel.

### Example 5.

A section of bowel from the pig in Example 4 was exposed and the MPH-thrombin/ albumin-fibrinogen preparations from Example 2 were applied. After application of the solutions an adherent gel coating of fibrin/MPH was formed over the bowel surface.

### Example 6.

The following three formulations were applied to a piece of fresh beef liver:
A. 0.015g MPH + 0.12g crosslinked hyaluronan (SepraGel Sinus, Genzyme)
B. 0.15g crosslinked hyaluronan (SepraGel Sinus, Genzyme)
C. 0.31g water + 0.53g crosslinked hyaluronan (SepraGel Sinus, Genzyme)

Formulation A was compared to formulation B on an angled surface of liver (i.e., almost vertical). Formulation A had better adhesion to the liver than formulation B. MPH was then sprayed onto a horizontal surface of liver until it stopped absorbing water (i.e. until the topmost layer stayed white). Formulation C was then sprayed onto the same horizontal surface, followed by another spray application of MPH. The layer thus formed completely covered and adhered to the application surface.

Liver with formulations A and B were immersed in saline. Traces could not be found after 5 min. soak. However, drops of saline placed on C did not dissolve the MPH/hyaluronan layer, but gave it a texture similar to that of a mucosal layer.

### Example 7

Platelet poor plasma was obtained by centrifuging citrated sheeps' blood. The supernatant was mixed with MPH by hand and physical consistency observed.

| **Ratio (ml plasma/g MPH)** | **Consistency** |
|---|---|
| 2 | Chunky, dry, not cohesive |
| 4 | Smoother, still not very cohesive |
| 5 | Almost cohesive, starting to achieve "peaking" like egg whites |
| 8 | Peaking, gel-like |
| 9 | Peaking, gel-like |
| 10 | Thinner, but still a gel |

Thus is can be seen that by mixing platelet rich plasma and MPH particles in the proper ratios, gels can be formed without the addition of thrombin. Such gels are desirable when applying platelet rich plasma to wound surfaces.

### Example 8:

Citrated sheeps' blood was mixed with MPH by hand and physical consistency observed.

| **Ratio (ml blood/g MPH)** | **Consistency** |
|---|---|
| Blood only | Liquid, not coagulated on plastic tray |
| 5 | Peaking, strong gel |
| 10 | Peaking, weaker gel |

As seen by these examples, the materials can be applied as fine sprays that can be applied into difficult to reach area of the bowel or to rapidly cover large exposed surfaces of tissue. The preparations can be prepared as flowable mixtures that quickly gel and adhere to the surface. Additional materials incorporated into the particle matrix or the liquid polymer solution can affect additional changes in the newly formed gel. For example, the serum albumin/MPH gels of Example 2 can be stabilized by entrapment into a fibrin matrix formed from fibrinogen in the albumin solution interacting with thrombin diffusing from the MPH particles as demonstrated in Example 3. Also in Example 1, the sodium alginate films gelled by the action of MPH particles can subsequently react with calcium ions released from the particles to form insoluble gels with a longer residence time in tissue than the initial gel. This ability to form altered gel films by reaction of materials incorporated into the two solutions can be used to create films with varying properties and is a useful feature of the invention. A wide variety of possible secondary reactions can be accomplished by proper choice of materials. The particles can be derivatized with a variety of reactive groups such as amino, carbonyl, or carboxyl. Complementary reactive groups in the polymer materials can react to form ionic complexes, Schiff bases, or similar stabilizing bonds.

The dry particles can also be used as carriers for cross-linking reagents that may be used to immobilize the polymer gels once formed. The gel formed by the combination of particles and polymer solution forms a concentrated reaction boundary at the interface between the particle and the polymer solution. This will increase reaction rates, thus forming an instantaneous gel using chemistries which would normally take longer to react.

## Claims

1. Composition for use in treatment of wounds or trauma of a surface of tissue of a patient, the composition being applied to the surface of tissue so that both a) a gel-forming composition comprising a solution, suspension, dispersion or emulsion and b) dry porous dextran or starch microparticles having pore sizes for molecular weight cutoff of between 5,000 Daltons and 200,000 Daltons are applied to the surface.

2. Composition for use in a treatment of claim 1, the treatment comprising forming a layer on a surface of tissue of a patient and the composition being applied to the surface of tissue so that both a) a gel- forming composition comprising a solution, suspension, dispersion or emulsion and b) dry porous dextran or starch microparticles are applied simultaneously or alternatively to the surface.

3. Composition for use in a treatment of claim 1 or 2 wherein the gel-forming composition is a hydrogel- forming composition and the hydrogel is applied as a first liquid composition and the dry porous dextran or starch microparticles are applied as a second composition.

4. Composition for use in a treatment of claim 3 wherein the dry porous dextran or starch microparticles are applied as a dry composition.

5. Composition for use in a treatment of claim 3 wherein at least one of the first composition and the second composition are applied by spraying.

6. Composition for use in a treatment of claim 1 or 2 wherein the gel-forming composition comprises at least one ingredient selected from the group consisting of
a) thrombin, b) albumin- fibrinogen, c) hyaluronan, d) cellulosic polymer, e) acrylic polymer and f) hydrophilic and crosslinkable polymer, and both the first composition and the second composition are applied by spraying.

7. Composition for use in a treatment of claim 4 wherein both the first composition and the second composition are applied by spraying.

8. Composition for use in a treatment of claim 3 wherein the first composition is applied at the same time as or before application of the second composition.

9. Composition for use in a treatment of claim 3 wherein the second composition is applied before application of the first composition.

10. An applicator system for application of the composition for use in a treatment of any one of claims 1 to 9, to tissue surfaces comprising: a first source of the gel or hydrogel forming composition; and a second source of the dry porous dextran or starch microparticles, a first conveying system for conveying said gel or hydrogel forming composition; and a second conveying system for conveying the dry porous dextran or starch microparticles, and a first applicator system for applying the gel or hydrogel forming composition to a surface, and a second applicator system for applying the dry porous dextran or starch microparticles to a surface.

11. The system of claim 10 wherein both of the gel or hydrogel composition is provided as a liquid.

12. The system of claim 11 wherein the first applicator system comprises a spray system.

13. The system of claim 12 wherein the second applicator system comprises a spray system.

14. Barrier layer adhered to a surface of a patient tissue comprising a composition for use in treatment of any one of claims 1 to 9.

15. Composition for use in a treatment of claim 1 or 2 wherein the dry microporous dextran or starch particles have an effective pore volume of 2% to 75% of the total volume of the microporous particles.

16. Composition for use in a treatment of claims 1 or 2 wherein the dry microporous dextran or starch particles are combined with a crosslinking agent for the gel-forming composition prior to contact with the gel-forming composition.

17. Composition for use in a treatment of claim 16 wherein contacting of the gel-forming composition and the microporous particles with crosslinking agent occurs on the surface of tissue to be treated.

## Patentansprüche

1. Zusammensetzung für die Verwendung bei der Behandlung von Wunden oder Trauma einer Oberfläche von Gewebe eines Patienten, wobei die Zusammensetzung so auf die Oberfläche von Gewebe aufgebracht wird, dass sowohl a) eine Gel-bildende Zusammensetzung, die eine Lösung, Suspension, Dispersion oder Emulsion umfasst, als auch b) trockene poröse Dextran- oder Stärke-Mikropartikel mit Porengrößen für eine Molekulargewichtsgrenze von zwischen 5.000 Dalton und 200.000 Dalton auf die Oberfläche aufgebracht werden.

2. Zusammensetzung für die Verwendung bei einer Behandlung gemäß Anspruch 1, wobei die Behandlung Bilden einer Schicht auf einer Oberfläche von Gewebe eines Patienten umfasst und die Zusammensetzung so auf die Oberfläche von Gewebe aufgebracht wird, dass sowohl a) eine Gel-bildende Zusammensetzung, die eine Lösung, Suspension, Dispersion oder Emulsion umfasst, als auch b) trockene poröse Dextran- oder Stärke-Mikropartikel gleichzeitig oder abwechselnd auf die Oberfläche aufgebracht werden.

3. Zusammensetzung für die Verwendung bei einer Behandlung gemäß Anspruch 1 oder 2, wobei die Gel-bildende Zusammensetzung eine Hydrogel-bildende Zusammensetzung ist und das Hydrogel als eine erste flüssige Zusammensetzung aufgebracht wird und die trockenen porösen Dextran- oder Stärke-Mikropartikel als eine zweite Zusammensetzung aufgebracht werden.

4. Zusammensetzung für die Verwendung bei einer Behandlung gemäß Anspruch 3, wobei die trockenen porösen Dextran- oder Stärke-Mikropartikel als eine trockene Zusammensetzung aufgebracht werden.

5. Zusammensetzung für die Verwendung bei einer Behandlung gemäß Anspruch 3, wobei wenigstens eine von der ersten Zusammensetzung und der zweiten Zusammensetzung durch Sprühen aufgebracht werden.

6. Zusammensetzung für die Verwendung bei einer Behandlung gemäß Anspruch 1 oder 2, wobei die Gel-bildende Zusammensetzung wenigstens einen Inhaltsstoff ausgewählt aus der Gruppe bestehend aus a) Thrombin, b) Albumin-Fibrinogen, c) Hyaluronan, d) Cellulosepolymer, e) Acrylpolymer und f) hydrophilem und vernetzbarem Polymer umfasst und sowohl die erste Zusammensetzung als auch die zweite Zusammensetzung durch Sprühen aufgebracht werden.

7. Zusammensetzung für die Verwendung bei einer Behandlung gemäß Anspruch 4, wobei sowohl die erste Zusammensetzung als auch die zweite Zusammensetzung durch Sprühen aufgebracht werden.

8. Zusammensetzung für die Verwendung bei einer Behandlung gemäß Anspruch 3, wobei die erste Zusammensetzung zugleich mit oder vor dem Aufbringen der zweiten Zusammensetzung aufgebracht wird.

9. Zusammensetzung für die Verwendung bei einer Behandlung gemäß Anspruch 3, wobei die zweite Zusammensetzung vor dem Aufbringen der ersten Zusammensetzung aufgebracht wird.

10. Applikatorsystem zum Aufbringen der Zusammensetzung für die Verwendung bei einer Behandlung gemäß einem der Ansprüche 1 bis 9 auf Gewebeoberflächen, umfassend: eine erste Quelle der Gel- oder Hydrogel-bildenden Zusammensetzung; und eine zweite Quelle der trockenen porösen Dextran- oder Stärke-Mikropartikel; ein erstes Fördersystem zum Befördern der Gel- oder Hydrogel-bildenden Zusammensetzung; und ein zweites Fördersystem zum Befördern der trockenen porösen Dextran- oder Stärke-Mikropartikel; und ein erstes Applikatorsystem zum Aufbringen der Gel- oder Hydrogel-bildenden Zusammensetzung auf eine Oberfläche; und ein zweites Applikatorsystem zum Aufbringen der trockenen porösen Dextran- oder Stärke-Mikropartikel auf eine Oberfläche.

11. System gemäß Anspruch 10, wobei beide von der Gel- oder Hydrogel-Zusammensetzung als Flüssigkeit bereitgestellt sind.

12. System gemäß Anspruch 11, wobei das erste Applikatorsystem ein Sprühsystem umfasst.

13. System gemäß Anspruch 12, wobei das zweite Applikatorsystem ein Sprühsystem umfasst.

14. Barriereschicht, die an einer Oberfläche eines Patientengewebes haftet, umfassend eine Zusammensetzung für die Verwendung bei der Behandlung gemäß einem der Ansprüche 1 bis 9.

15. Zusammensetzung für die Verwendung bei einer Behandlung gemäß Anspruch 1 oder 2, wobei die trockenen mikroporösen Dextran- oder Stärke-Mikropartikel ein effektives Porenvolumen von 2 % bis 75 % des Gesamtvolumens der mikroporösen Partikel aufweisen.

16. Zusammensetzung für die Verwendung bei einer Behandlung gemäß Anspruch 1 oder 2, wobei die trockenen mikroporösen Dextran- oder Stärke-Mikropartikel vor dem Kontakt mit der Gel-bildenden Zusammensetzung mit einem Vernetzungsmittel für die Gel-bildende Zusammensetzung kombiniert werden.

17. Zusammensetzung für die Verwendung bei einer Behandlung gemäß Anspruch 16, wobei Inkontaktbringen der Gel-bildenden Zusammensetzung und der mikroporösen Partikel mit Vernetzungsmittel auf der Oberfläche von zu behandelndem Gewebe erfolgt.

## Revendications

1. Composition pour utilisation dans le traitement de plaies ou de traumatisme d'une surface de tissu d'un patient, la composition étant appliquée sur la surface de tissu de sorte que a) une composition formant un gel comprenant une solution, suspension, dispersion ou émulsion et b) des microparticules poreuses sèches de dextrane ou d'amidon ayant des tailles de pore pour un seuil de coupure de poids moléculaire compris entre 5 000 daltons et 200 000 daltons soient appliquées sur la surface.

2. Composition pour utilisation dans un traitement de la revendication 1, le traitement comprenant la formation d'une couche sur une surface de tissu d'un patient et la composition étant appliquée sur la surface de tissu de sorte que a) une composition formant un gel comprenant une solution, suspension, dispersion ou émulsion et b) des microparticules poreuses sèches de dextrane ou d'amidon soient appliquées simultanément ou alternativement sur la surface.

3. Composition pour utilisation dans un traitement de la revendication 1 ou 2 où la composition formant un gel est une composition formant un hydrogel et l'hydrogel est appliqué sous la forme d'une première composition liquide et les microparticules poreuses sèches de dextrane ou d'amidon sont appliquées en tant que deuxième composition.

4. Composition pour utilisation dans un traitement de la revendication 3 où les microparticules poreuses sèches de dextrane ou d'amidon sont appliquées sous la forme d'une composition sèche.

5. Composition pour utilisation dans a traitement de la revendication 3 où au moins l'une de la première composition et la deuxième composition est appliquée par pulvérisation.

6. Composition pour utilisation dans a traitement de la revendication 1 ou 2 où la composition formant un gel comprend au moins un composant choisi dans le groupe constitué de a) la thrombine, b) l'albumine-fibrinogène, c) le hyaluronane, d) un polymère cellulosique, e) un polymère acrylique et f) un polymère hydrophile et réticulable, et la première composition et la deuxième composition sont appliquées par pulvérisation.

7. Composition pour utilisation dans un traitement de la revendication 4 où la première composition et la deuxième composition sont appliquées par pulvérisation.

8. Composition pour utilisation dans un traitement de la revendication 3 où la première composition est appliquée en même temps que ou avant l'application de la deuxième composition.

9. Composition pour utilisation dans un traitement de la revendication 3 où la deuxième composition est appliquée avant l'application de la première composition.

10. Système d'applicateur pour application de la composition pour utilisation dans un traitement de l'une quelconque des revendications 1 à 9, sur des surfaces de tissu comprenant : une première source de la composition formant un gel ou un hydrogel ; et une deuxième source des microparticules poreuses sèches de dextrane ou d'amidon, un premier système de transport pour transporter ladite composition formant un gel ou un hydrogel ; et un deuxième système de transport pour transporter les microparticules poreuses sèches de dextrane ou d'amidon, et un premier système d'applicateur pour appliquer la composition formant un gel ou un hydrogel sur une surface, et un deuxième système d'applicateur pour appliquer les microparticules poreuses sèches de dextrane ou d'amidon sur une surface.

11. Système de la revendication 10 dans lequel la composition de gel ou d'hydrogel est fournie sous la forme d'un liquide.

12. Système de la revendication 11 dans lequel le premier système d'applicateur comprend un système de pulvérisation.

13. Système de la revendication 12 dans lequel le deuxième système d'applicateur comprend un système de pulvérisation.

14. Couche de barrière adhérant à une surface d'un tissu de patient comprenant une composition pour utilisation dans le traitement de l'une quelconque des revendications 1 à 9.

15. Composition pour utilisation dans un traitement de la revendication 1 ou 2 où les particules microporeuses sèches de dextrane ou d'amidon ont un volume de pore effectif de 2 % à 75 % du volume total des particules microporeuses.

16. Composition pour utilisation dans un traitement des revendications 1 ou 2 où les particules microporeuses sèches de dextrane ou d'amidon sont combinées avec un agent de réticulation pour la composition formant un gel avant contact avec la composition formant un gel.

17. Composition pour utilisation dans a traitement de la revendication 16 où la mise en contact de la composition formant un gel et des particules microporeuses avec l'agent de réticulation se produit sur la surface de tissu devant être traitée.
